# EUROPEAN PATENT SPECIFICATION

(11) **EP 3 410 991 B1**
(45) Date of publication and mention of the grant of the patent: **19.11.2025**
(21) Application number: 17704919.4
(22) Date of filing: 31.01.2017
(51) Int. Cl.: A61F 5/44, A61F 5/445

(54) **OSTOMY POUCH AND OSTOMY POUCH OUTLET**
OSTOMIEBEUTEL UND OSTOMIEBEUTELAUSLASS
POCHE POUR STOMIE ET SORTIE DE POCHE POUR STOMIE

(30) Priority: 05.02.2016 US 201662291969 P
(43) Date of publication of application: 12.12.2018
(73) Proprietor: Hollister Incorporated, Libertyville, IL 60048 (US)
(72) Inventor: GRUM-SCHWENSEN, Christen, Libertyville, IL 60048 (US); TORSTENSEN, Jan, Libertyville, IL 60048 (US); MAACK, Mette, Dybendal, Libertyville, IL 60048 (US); NIELSEN, Kenneth, Libertyville, IL 60048 (US); RICHMANN, Sussie, Libertyville, IL 60048 (US); WOHLGEMUTH, Jan, Libertyville, IL 60048 (US); MOLLER-JENSEN, Peter, Libertyville, IL 60048 (US); SKJODT, Ole, Libertyville, IL 60048 (US)
(74) Representative: FRKelly
(86) International application number: PCT/US2017/015763
(87) International publication number: WO 2017/136314

(56) References cited:
- EP-A1- 3 410 992
- WO-A1-01/28470
- WO-A2-2006/031275
- US-A- 3 825 005
- US-A- 4 238 059
- US-A- 4 486 923
- US-A1- 2012 138 623
- US-A1- 2013 253 456

## Description

### BACKGROUND

The following description generally relates to ostomy appliances, and in particular, an ostomy pouch having a pouch outlet with a closure member.

Ostomy pouches for collecting bodily waste are used by patients who have had surgery such as a colostomy, ileostomy, or urostomy. Ostomy pouches typically include flat, opposing side walls secured together along their edges to define a collection cavity. One of the side walls is provided with an opening to receive a stoma, and means to secure the pouch to the user, such as an adhesive barrier, so that body waste discharged through the stoma is received within the cavity without leakage from the stoma/barrier/pouch environment.

The ostomy pouch may be a drainable pouch having a discharge opening at a lower end. The discharge opening may be closed during collection of body waste material, but may be opened to drain the waste material from the pouch. Such drainable ostomy pouches are disclosed, for example, in Nolan, U.S. Pat. No. 3,523,534, and Jensen et al., U.S. Pat. No. 4,411,659.

US 3,825,005 discloses a reusable resealable ileostomy or colostomy bag having ribbed resilient overlapping panels of plastics material.

The discharge opening of drainable ostomy pouches is typically defined at the end of a narrowed neck portion, which is provided with closure means for maintaining the discharge opening in a sealed condition until waste material is to be drained from the pouch. The closure means may take the form of a clamp, as in the aforementioned Nolan patent, or a device such as conventional wire tires or wraps for securing the neck portion in an upwardly-rolled condition.

However, the discharge openings described above may be difficult to manufacture or use. For example, in an ostomy pouch that includes a clamp for closing the discharge opening, the clamp is typically manufactured separately and attached to the pouch in a post-manufacturing step. In addition, a discharge opening or neck portion having a discharge opening of an ostomy pouch may need to be designed and manufactured with a specific geometry to be structurally and functionally compatible with the clamp. Further, a neck portion that is closed by upward rolling and tying or wrapping may be difficult to use for patients having limited dexterity. Further still, traditional upward-rolling closures cannot be attached to a night drainage system.

Accordingly, it is desirable to provide a drainable ostomy pouch having an outlet that may be easily manufactured and used.

US4238059 (A) discloses a stoma drainage appliance having a fluid collecting pouch constructed of sheets of flexible material with two internal sheets sealed together at spaced intervals to provide a valve across the center of the pouch precluding upward flow of fluid between the two internal sheets. There is at least one unsealed space between the seals located so as to provide a direct, straight path from an inlet opening of the upper section of the pouch to the lower section of the pouch. The stoma drainage appliance further includes an outlet spout connected at the lower section of the pouch. The spout has a tapered opening with an inlet neck section of oval cross-section and an outlet end section of circular cross-section. The spout is made of flexible material for clamping along the neck section to close the spout and includes a cap for attachment to the end section thereof. A tubular coupling member is provided for connecting to the spout and it has an opening of tapered construction to accommodate external conduits of varying size.

WO2006031275 (A2) discloses a drainable pouch having sidewalls of flexible sheet material which define a body portion having a cavity therein. The body portion includes a discharge end provided with a compliant internal adapter forming a discharge opening for draining the contents of the pouch. The pouch also includes a two-part external fastener system for closing and opening the discharge opening formed by the compliant internal adapter including first and second fastener parts having locking elements that can be brought into releasable engagement when the discharge end of the pouch is folded. The first and second fastener parts cooperate with the compliant internal adapter to cause the discharge opening to be maintained in a closed position after the locking elements have been brought into releasable engagement by folding the discharge end of the pouch. The pouch is thereafter drainable by releasing the locking elements of the first and second fastener parts from engagement and unfolding the discharge end of the pouch to open the discharge opening formed by the compliant internal adapter for draining the pouch through the discharge opening.

US2013253456 (A1) discloses a drainable ostomy pouch includes a closure system, which includes four closure members and a two-part fastening system. The closure system is arranged on a neck portion of the drainable pouch, and configured such that the pouch can be securely sealed after the neck portion is folded up three times and closed using the two-part fastening system.

WO0128470 (A1) discloses a collecting bag comprising a bag member (301) formed by two film blanks with joined edges and an inlet opening for connection of the bag to a body orifice provided in one of said film blanks, an elongated discharge portion (308) ending in a discharge opening (309) is connected with the bag member, said discharge portion having a width permitting opening and reclosing by manual unfolding and folding operations. A narrowed discharge passage (337) is defined in the discharge portion (308) for the discharge of more or less entirely liquid body wastes and has a width less than 60 % of the minimum width of the discharge portion (308).

US4486923 (A) discloses a closure device for a surgical post-operative drainage bag comprises a first panel which is curved and a second panel. The two panels are joined by a hinged connection. The second panel comprises two strip-like portions joined together by an intermediate hinge. When the device is closed, the free edge of the second panel tightly engages within the inside of the lip so as to be placed in a stressed condition and so that a portion of the bag positioned between the inside of the lip and the free edge of the second panel may be secured.

US2012138623 (A1) discloses a system and method for providing a reclosable snap closure. One embodiment relates to a closure device, including at least one pivot member, a first member and a second member. The first member has a predefined length LS and is pivotally connected to the at least one pivot member. The second member has a predefined length LF such that LF>=LS and is pivotally connected to the at least one pivot member and movably engages the first member, the first member creating a spring load, which is transferred through the at least one pivot member to at least the second member, thereby providing a relatively high pressure along a portion of at least the second member engaging the first member.

### SUMMARY

The present disclosure provides an ostomy pouch as detailed in claim 1. Advantageous features are provided in dependent claims.

According to one embodiment, there is provided an ostomy pouch having a collection pouch with a first side wall and a second side wall connected to one another along a periphery, the collection pouch having a collection cavity defined between the first and second side walls, an outlet device connected to and extending from the collection pouch, the outlet device formed separately from the collection pouch and attached to a collection pouch outlet and defining an internal passageway connected to the collection cavity, and a closure member on the outlet device configured to selectively close the outlet device. The outlet device may be injection molded or blow molded as a single unit and sealed to the collection pouch in a single processing step.

According to another embodiment there is provided an ostomy pouch having a collection pouch defining a collection cavity therein and having an opening configured to receive a stoma, a separate outlet device extending from and sealed to the collection pouch, the outlet device defining an internal passageway connected to the collection cavity, and a closure member on the outlet device configured to selectively close the outlet device.

According to still another embodiment, there is provided an ostomy pouch including a collection pouch having a collection cavity defined therein and an opening configured to receive a stoma, an outlet device extending from and sealed to the collection pouch, the outlet device defining an internal passageway connected to the collection cavity, a closure member on the outlet device, the closure member comprising a single fold line and a releasable fastener, and a drainage attachment connected to the outlet device, the drainage attachment having an internal connecting passageway connected to the internal passageway, a first segment for engaging the outlet device, a second segment configured for connection to a drainage system, and a securing strap configured to releasable engage the closure member.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a perspective view of an ostomy pouch according to an embodiment described herein;
FIG. 2 shows a closure member for an ostomy pouch according to an embodiment described herein;
FIG. 3 is a perspective view of an ostomy pouch outlet according to another embodiment described herein;
FIG. 4 is another perspective view of the ostomy pouch outlet of FIG. 3;
FIG. 5 is a perspective view showing a closure member on the outlet of FIGS. 3 and 4, according to an embodiment described herein;
FIG. 6 is a perspective view of an ostomy pouch having an outlet according to another embodiment described herein;
FIG. 7 is a perspective view of an ostomy pouch having the outlet of FIG. 6 in an open condition, according to an embodiment described herein;
FIG. 8 is a perspective view of an ostomy pouch having the outlet of FIG. 6 connected to a night drainage system according to an embodiment described herein;
FIG. 9 is a perspective view of a first side of an ostomy pouch having an outlet according to yet another embodiment described herein;
FIG. 10 is a perspective view of a second side of the outlet of FIG. 9;
FIG. 11 is a perspective view of the outlet of FIG. 9 in a folded up position;
FIG. 12 is a perspective view of the outlet of FIG. 9 in a folded up and closed position; and
FIG. 13 is a perspective view of a drainable attachment according to an embodiment described herein; and
FIG. 14 is a side perspective view of the drainable attachment of FIG. 13.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

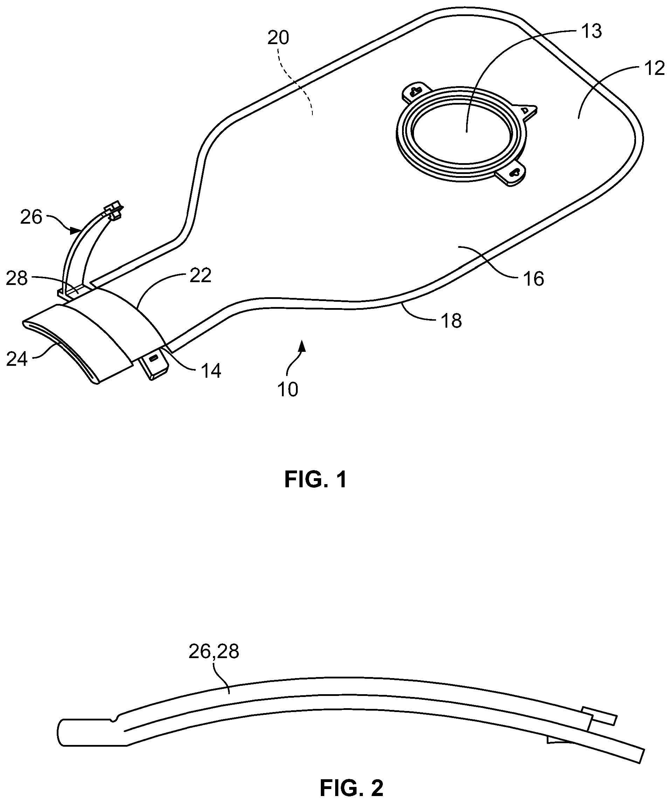

Referring generally to FIGS. 1-8, the embodiments described herein include an ostomy pouch 10 having a collection pouch 12 and an outlet 14. The collection pouch 12 may generally be formed by a first side wall 16 and a second side wall 18 joined to one another at a periphery, or connected integrally with one another as a single unit. A collection cavity 20 is defined within the collection pouch 12, i.e., between the first and second side walls 16, 18. The collection pouch 12 also includes an inlet 13 formed in the first side walls 16 configured to receive a stoma so that a discharge from the stoma may be received in the collection cavity 20. The inlet 13 may be positioned at an upper portion of the collection pouch 12. The collection pouch 12 further includes a discharge port 22 configured for connection to the outlet 14. The discharge port 22 may be, for example, an opening formed in the periphery or one of the side walls 16, 18 and may be disposed at a lower portion of the collection pouch 12.

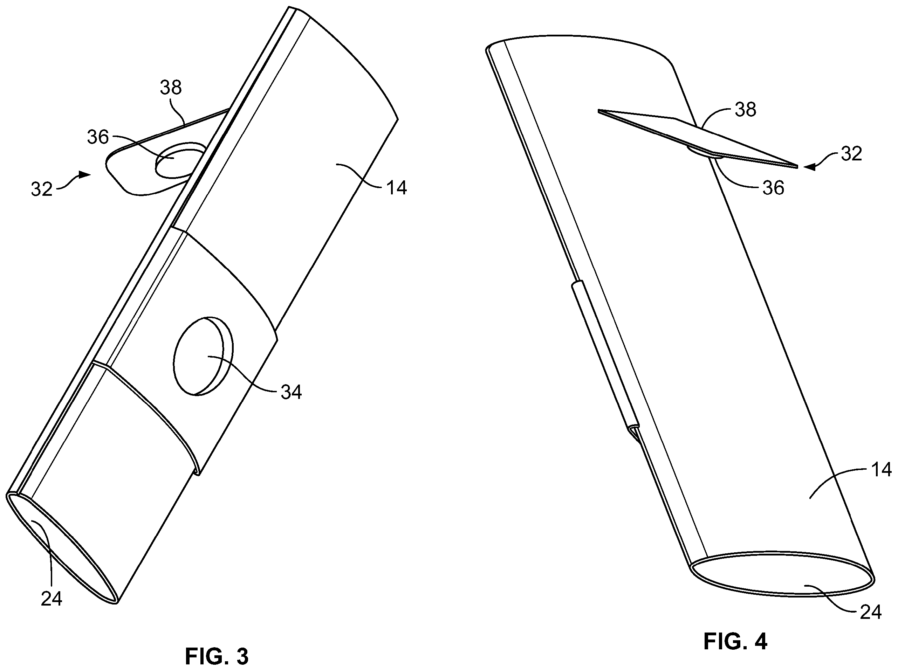

In one embodiment, the outlet 14 may be formed as a single, continuous part, for example, in a molding process. For example, in one embodiment, the outlet 14 may be injection molded. In another embodiment, the outlet 14 may be blow molded. The outlet 14 includes an internal passageway 24 (see, for example, FIGS. 3-6 and 8) connected to the collection cavity 20 so that contents may flow from the collection cavity 20 into the internal passageway 24 via the discharge port 22. In one embodiment, the outlet 14 may be formed separately from the collection pouch 12 and connected to the collection pouch 12 at the discharge port 22. The outlet 14 may be connected to the collection pouch 12 in any suitable manner that seals the outlet 14 to the collection pouch 12 such that contents may flow from the collection cavity 20 to the internal passageway 24 without leaking. For example, the outlet 14 may be heat sealed, welded, or the like, to the collection pouch 12. In other embodiments, the outlet 14 may be formed integrally and continuously with the collection pouch 12.

The ostomy pouch 10 also includes a closure member 26. The closure member 26 is configured to close and/or seal the outlet 14 during normal daily use of the ostomy pouch 10. That is, the closure member 26 may be moved to a closed condition where the internal passageway 24 of the outlet 14 is closed to limit or prevent inadvertent leakage of the contents from the outlet 14 during daily use.

In some embodiments, the closure member 26 is formed on the outlet 14. The closure member 26 may be integrated with the outlet 14 as a single unit. In one embodiment, the closure member 26 may be, for example, a clamp, such as a bistable clamp configured to selectively close and seal the internal passageway 24. In other embodiments, the closure member 26 may be formed by one or more folds or fold lines in the outlet 14, where the outlet 14 may be folded onto itself. The closure member 26 may also include a releasable fastener to hold the outlet in a closed condition. The releasable fastener may be, for example, a snap, a hook and loop fastener, or other similar, suitable fasteners.

Referring to FIGS. 1 and 2, in one embodiment, the outlet 14 may be formed separately from the collection pouch 12. For example, the outlet 14 may be formed as a single, continuous unit in an injection molding process and secured to the collection pouch 12 such that the collection cavity 20 is connected to the internal passageway 24 to allow for flow of contents from the collection cavity 20 to the internal passageway 24. Alternatively, the outlet 14 may be formed of several components secured together, and then secured to the collection pouch 12. In one embodiment, the outlet 14 may be sealed to the collection pouch 12, for example, by heat sealing or welding in a single process step.

The closure member 26, may be, for example a clamp 28. In one embodiment, the clamp 28 may be a bistable clamp movable between a first stable position and a second stable position. In the first stable position, the clamp 28 is curved and closed, to seal the internal passageway 24 of the outlet 14. In the second stable position, the clamp 28 is open. In one embodiment, the clamp 28 may be secured to the outlet 14 by way of a second integrated clamp, formed with the outlet 14. In another embodiment, the clamp 28 is formed integrally and continuously with the outlet 14 in the injection molding process, so that the outlet 14 and closure member 26 may be secured to the collection pouch 12 in a single processing step.

Implementation of the clamp 28 as the closure member, as opposed to, for example, upward folding or rolling of a pouch neck, may allow the outlet 14 be more easily used by those having limited dexterity. In addition, because the outlet 14 is made separately from the collection pouch 12, and in an injection molding process, the outlet 14 may be mass produced and costs may be reduced.

FIGS. 3-5 show a variation of the outlet 14 described herein. In this embodiment, the outlet 14 may be formed, for example, from blow molding. Accordingly, the outlet 14 may be formed substantially as a tube or tube-like section. In one embodiment, the outlet 14 may be made in what is understood to those having skill in the art as a 2K molding to improve properties. The outlet 14 may be made from a suitable polymeric material. As described above, the outlet 14 may then be secured to the collection pouch 12, for example, by heat sealing, welding or similar sealing fastening technique.

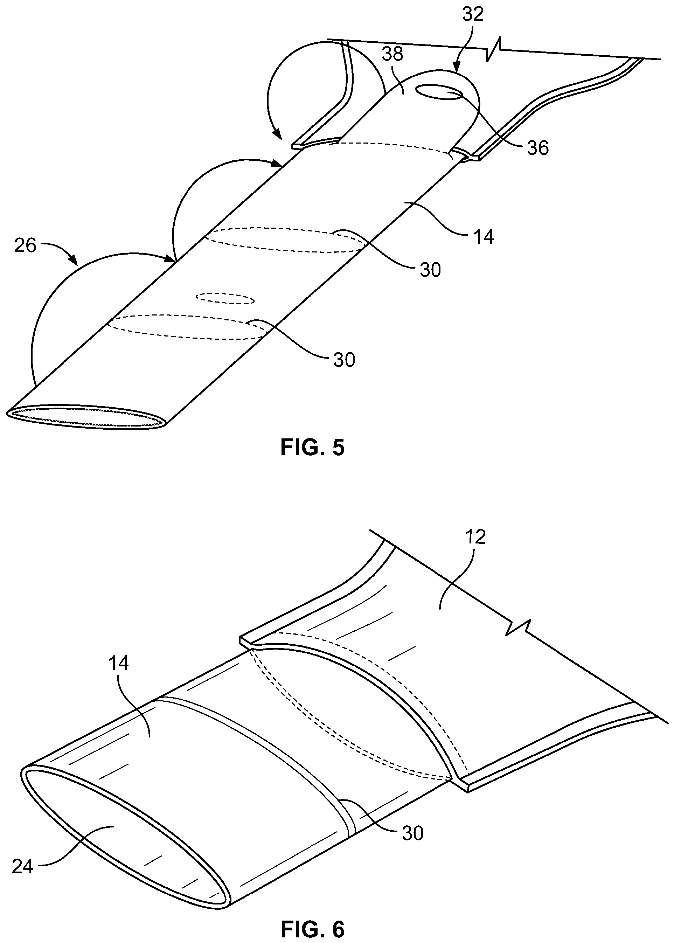

Referring still to the FIGS. 3-5, and in particular, FIG. 5, the closure member 26 may be integrated into the outlet 14 as one or more fold lines 30 and a releasable fastener 32. In one embodiment, the closure member 26 may be implemented as two fold lines 28 at which the outlet 14 may be folded over on itself to close or seal the internal passageway 24. The releasable fastener 32 may be formed in its entirety on the outlet 14. Accordingly, the outlet 14 and the closure member 26 may be secured to the collection pouch 12 in a single process step.

The releasable fastener 32 is configured to hold the outlet 14 in a closed condition, i.e., hold the outlet 14 in the condition where it is folded on itself at the fold lines 30. The releasable fastener 32 may be, for example, a hook and loop fastener, a snap, or the like. In one embodiment, the releasable fastener 32 may be implemented as a snap having a first snap part 34 at a first position on the outlet 14, and a second, mating snap part 36 at a second position on the outlet 14. In one embodiment, the second snap part 36 may be positioned on a flexible tab 38 that may be folder over so the first snap part 34 and the second snap part 36 matingly engage when the outlet 14 is folded at the two fold lines 30. It is understood that other releasable fasteners, such as a hook and loop fastener, may be similarly implemented. The outlet 14 may be moved to an open condition, where the collection pouch 12 may be drained, for example, by releasing the releasable fastener 32 and unfolding the outlet 14.

In the configuration shown in FIGS. 3-5, the outlet 14 may be easily produced as a single piece, i.e., an outlet 14 and closure member 26 for attachment to a collection pouch 12 in a single process step. Further, by including only two folds, and a releasable fastener, a user may easily operate the ostomy pouch 10 to move the outlet 14 between the open condition and the closed condition, and move the outlet 14 between the open and closed conditions by actuating the releasable fastener 32. Further, in some embodiments, a free end of the outlet 14 is configured to have a drainage connector or attachment secured thereto so that the ostomy pouch may be combined with a night drainage system, as discussed further below.

FIGS. 6-8 show another variation of the outlet 14 according to an embodiment described herein. In one embodiment, the outlet 14 may be sealingly secured or integrally connected to the collection pouch 12. The closure member 26 may also be integrated with outlet 14. The closure member 26 may be implemented as, for example, a fold line 30 and releasable fastener 32.

In one embodiment, the closure member 26 includes a single fold line 30 at which the outlet 14 may be folded over on itself to close or seal the internal passageway 24 of the outlet 14. The outlet 14 may be held in the closed condition by way of the releasable fastener 32. In one embodiment, the releasable fastener 32 may include a first snap member 34 and a second mating snap member 36. The first snap member 34 is formed on an opposite side of the fold line 30 from the second snap member 36. The first and second snap members 34, 36 are positioned so as to overlap when the outlet 14 is folded at the fold line 30. Accordingly, the first and second snap members 34, 36 releasably engage one another to hold the outlet 14 in the closed condition.

Conversely, to move the outlet 14 to the open condition, the releasable fastener 32 is released, and the outlet 14 is unfolded. It is understood that other suitable releasable fasteners are envisioned that may be operate in a similar manner, such as a hook and loop fastener.

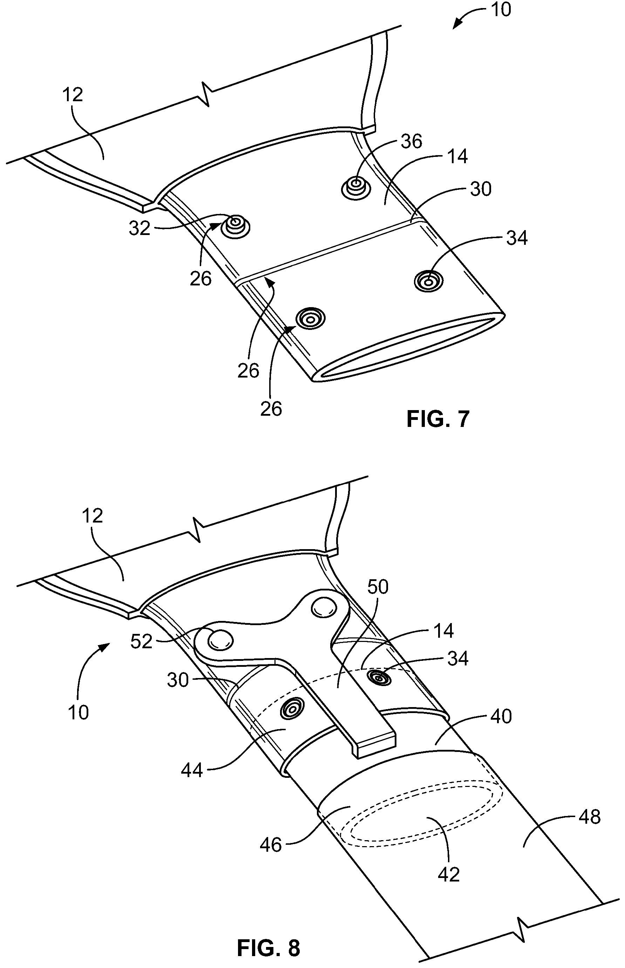

Referring to FIG. 8, in one embodiment, the outlet 14 has a drainage attachment 40 secured thereto. In one embodiment, the drainage attachment 40 may be formed as a substantially conical or frustoconical section having a connecting passageway 42 formed therein. A first segment 44 of the drainage attachment 40 may be received in the outlet 14 to secure the attachment 40 to the outlet 14, while a second segment 46 of the drainage attachment 40 extends outwardly from the outlet 14. Preferably, the drainage attachment 40 is secured to the outlet 14 in a sealed connection. Accordingly, contents may be received from the collection cavity 20 into the internal passageway outlet 14, and then into the connecting passageway 42 of the drainage attachment 40. A hose or tube 48 of a drainage system, for example, a night drainage system, may be secured to the drainage attachment, for example, at the second segment 46. Accordingly, contents may flow from the connecting passageway 42 of the drainage attachment 40 into the hose 48 of the drainage system, and subsequently, to a reservoir (not shown).

With further reference to FIG. 8, the drainage attachment 40 may further include a strap 50 for providing additional security to the attachment 40. In one embodiment, the strap 50 is configured to releasably engage the closure member 26 of the outlet 14, to further secure the drainage attachment 40 to the outlet 14. For example, the strap 50 may be formed having a releasable fastener, such as a third snap member 52 configured to engage the second snap member 36 on the outlet 14 with the outlet 14 in the open condition. It is understood that other releasable fasteners may be implemented on the strap 50 that are configured to matingly and releasably engage the closure member 26 of the outlet 14.

Accordingly, the outlet 14 may be closed and/or sealed by folding at the fold line 30, and opened by unfolding at the fold line 30. Additionally, the ostomy pouch 10 may be coupled to a drainage system, such as a night drainage system, by way of the drainage attachment 50 secured to the outlet 14.

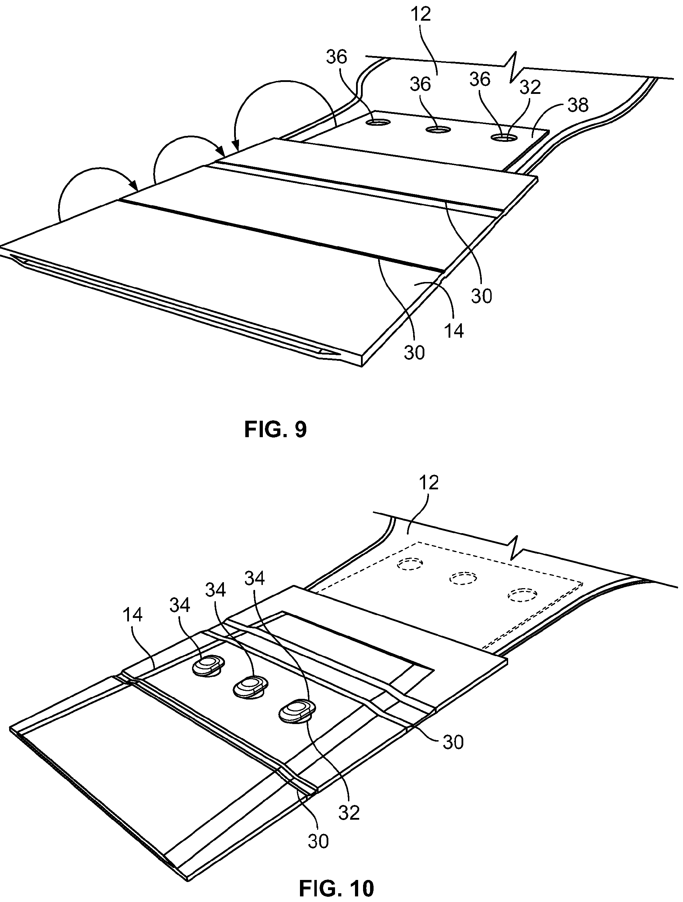

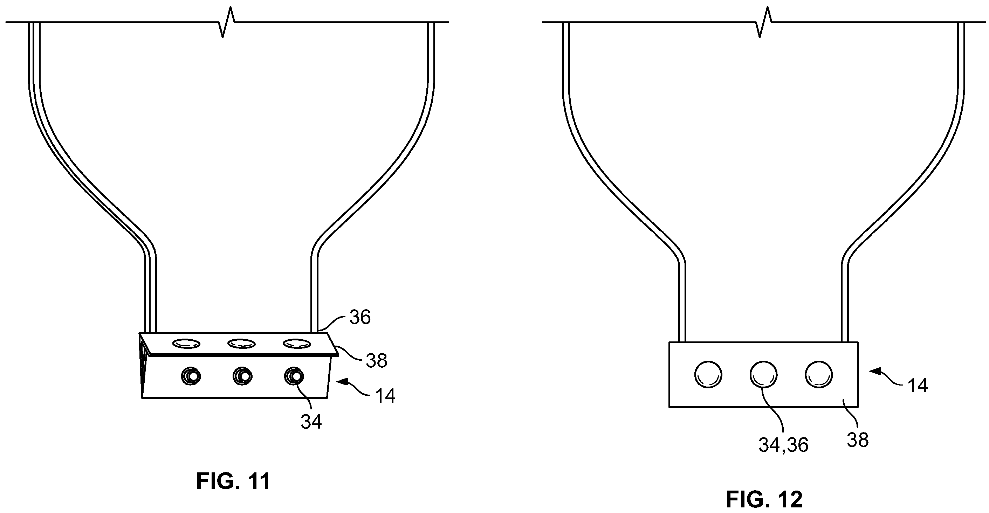

FIGS. 9-12 show another variation of the outlet 14 according to an embodiment described herein. In one embodiment, the outlet 14 may be sealingly secured or integrally connected to the collection pouch 12. In this embodiment, the closure member may comprise two fold lines 30 and a pair of releasable fasteners 32. The outlet 14 may be configured to be folded two times along the fold lines 30 in an upward direction as shown with arrows in FIG. 9 to close and seal the outlet 14. The outlet 14 may be held in the closed condition by way of the releasable fastener 32 as shown in FIGS. 11 and 12.

In the embodiment of FIGS. 9-12, the releasable fastener 32 may comprise a set of first snap members 34 and a set of second mating snap members 36. The set of first snap members 34 may be arranged at a first position on the outlet 14, and the set of second snap members 36 may be arranged at a second position on the outlet 14, such that the first and second snap members may be matingly engaged after the outlet 14 is folded two times. In an embodiment, the set of second snap members 36 may be provided on a flexible tab 38 that may be folder over so the set of first snap members 34 and the set of second snap members 36 may matingly engage when the outlet 14 is folded at the two fold lines 30. It is understood that other releasable fasteners, such as a hook and loop fastener, may be similarly implemented.

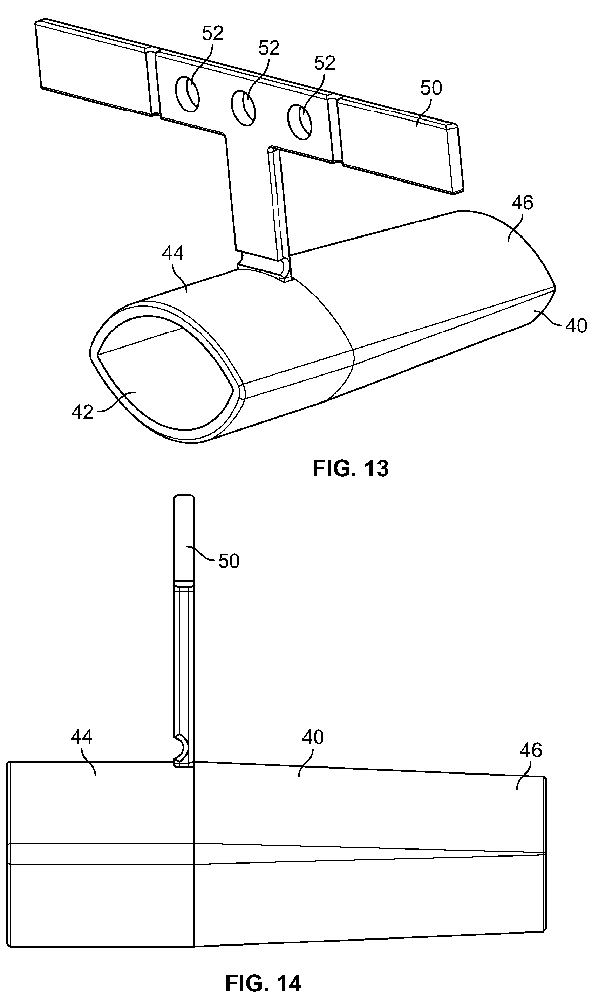

FIGS. 13 and 14 show a variation of a drainage attachment 40 according to an embodiment described herein. The drainage attachment 40 may be configured to connect with the outlet 12 of FIGS. 9-12 and formed as a substantially conical or frustoconical section having a connecting passageway 42 formed therein. A first segment 44 of the drainage attachment 40 may be received in the outlet 14 to secure the attachment 40 to the outlet 14, while a second segment 46 of the drainage attachment 40 extends outwardly from the outlet 14 in a similar configuration as the embodiment of FIG 8. A hose or tube of a drainage system, for example, a night drainage system, may be secured to the drainage attachment, for example, at the second segment 46.

The drainage attachment 40 may further include a strap 50 for providing additional security to the attachment 40. In one embodiment, the strap 50 may be configured to releasably engage the set of first snap members 34 of the outlet 14 of FIG 10, to further secure the drainage attachment 40 to the outlet 14. For example, the strap 50 may be formed having a releasable fastener, such as a set of third snap members 52 configured to engage the set of first snap members 34 on the outlet 14 with the outlet 14 in the open condition. It is understood that other releasable fasteners may be implemented on the strap 50 that are configured to matingly and releasably engage the closure member of the outlet 14.

It is understood that the features described with respect to any of the embodiments above may be implemented, used together with, or replace features described in any of the other embodiments above.

In the present disclosure, the words "a" or "an" are to be taken to include both the singular and the plural. Conversely, any reference to plural items shall, where appropriate, include the singular.

From the foregoing it will be observed that numerous modifications and variations can be effectuated without departing from scope of the novel concepts of the present invention. It is to be understood that no limitation with respect to the specific embodiments illustrated is intended or should be inferred. The disclosure is intended to cover by the appended claims all such modifications as fall within the scope of the claims.

## Claims

1. An ostomy pouch (10) comprising:
a collection pouch (12) having a collection cavity (20) defined therein and an opening (13) configured to receive a stoma;
an outlet device (14) connected to and extending from the collection pouch (12), the outlet device (14) defining an internal passageway (24) connected to the collection cavity (20), a closure arrangement (26) on the outlet device (14) configured to selectively close the outlet device (14), wherein the closure arrangement (26) comprises one or more fold lines (30) in the outlet device and a releasable fastener (32), wherein the outlet device (14) is configured to be folded onto itself along the one or more fold lines, and the releasable fastener (32) is configured to hold the outlet device (14) in a folded and closed condition, **characterised in that** the outlet device is formed separately from the collection pouch and attached to a collection pouch outlet, and **in that** the outlet device (14) and closure arrangement (26) are integrally formed as a single unit separate from the collection pouch (12).

2. The ostomy pouch (10) of claim 1, wherein the outlet device (14) is connected to the collection pouch outlet by heat sealing or welding, such that the outlet device (14) is sealed to the collection pouch outlet.

3. The ostomy pouch (10) of claim 1, wherein the outlet device (14) is injection molded as a single unit.

4. The ostomy pouch (10) of claim 1, wherein the outlet device (14) is blow molded.

5. The ostomy pouch (10) of any of the preceding claims, wherein the closure arrangement (26) comprises a single fold line (30) and the releasable fastener (32) is a snap (34).

6. The ostomy pouch (10) of any of the preceding claims, wherein the closure arrangement (26) includes two fold lines (30) and the releasable fastener (32) is one of a snap, hook and loop fastener, or a clip.

7. The ostomy pouch (10) of claim 1, further comprising a drainage attachment (40) having a first end configured to be received in the outlet device (14) and a second end configured for attachment to a drainage tube (48).

8. The ostomy pouch (10) of claim 7, wherein the drainage attachment (40) includes an at least partially conical section.

9. The ostomy pouch (10) of claim 7, further comprising a securing strap (50) on the drainage attachment (40) configured for connection to the closure arrangement (26).

10. The ostomy pouch (10) of claim 1, further comprising a drainage attachment (40) connected to the outlet device, the drainage attachment (40) having an internal connecting passageway (42) connected to the internal passageway (24), a first segment (44) for engaging the outlet device (14), a second segment (46) configured for connection to a drainage system (40), and a securing strap (50) configured to releasably engage the closure arrangement (26).

## Patentansprüche

1. Ostomiebeutel (10), umfassend:
einen Sammelbeutel (12), der einen darin definierten Sammelhohlraum (20) und eine Öffnung (13) aufweist, die dazu konfiguriert ist, ein Stoma aufzunehmen;
eine Auslassvorrichtung (14), die mit dem Sammelbeutel (12) verbunden ist und sich von diesem erstreckt, wobei die Auslassvorrichtung (14) einen inneren Durchgang (24) definiert, der mit dem Sammelhohlraum (20) verbunden ist, eine Verschlussanordnung (26) an der Auslassvorrichtung (14), die dazu konfiguriert ist, die Auslassvorrichtung (14) selektiv zu verschließen, wobei die Verschlussanordnung (26) eine oder mehrere Faltlinien (30) in der Auslassvorrichtung und ein lösbares Befestigungselement (32) umfasst, wobei die Auslassvorrichtung (14) dazu konfiguriert ist, entlang der einen oder mehreren Faltlinien auf sich selbst gefaltet zu werden, und das lösbare Befestigungselement (32) dazu konfiguriert ist, die Auslassvorrichtung (14) in einem gefalteten und verschlossenen Zustand zu halten, **dadurch gekennzeichnet, dass** die Auslassvorrichtung getrennt von dem Sammelbeutel gebildet und an einem Sammelbeutelauslass angebracht ist, und dass die Auslassvorrichtung (14) und die Verschlussanordnung (26) einstückig als eine einzige Einheit getrennt von dem Sammelbeutel (12) ausgebildet sind.

2. Ostomiebeutel (10) nach Anspruch 1, wobei die Auslassvorrichtung (14) mit dem Auslass des Sammelbeutels durch Heißsiegeln oder Schweißen derart verbunden ist, dass die Auslassvorrichtung (14) mit dem Auslass des Sammelbeutels versiegelt ist.

3. Ostomiebeutel (10) nach Anspruch 1, wobei die Auslassvorrichtung (14) als eine einzige Einheit spritzgegossen ist.

4. Ostomiebeutel (10) nach Anspruch 1, wobei die Auslassvorrichtung (14) blasgeformt ist.

5. Ostomiebeutel (10) nach einem der vorhergehenden Ansprüche, wobei die Verschlussanordnung (26) eine einzige Faltlinie (30) umfasst und das lösbare Befestigungselement (32) ein Druckknopf (34) ist.

6. Ostomiebeutel (10) nach einem der vorhergehenden Ansprüche, wobei die Verschlussanordnung (26) zwei Faltlinien (30) beinhaltet und das lösbare Befestigungselement (32) eines von einem Druckknopf, einem Klettverschluss oder einer Klammer ist.

7. Ostomiebeutel (10) nach Anspruch 1, ferner umfassend eine Drainagebefestigung (40), die ein erstes Ende, das dazu konfiguriert ist, in der Auslassvorrichtung (14) aufgenommen zu werden, und ein zweites Ende, das zur Befestigung an einem Drainageschlauch (48) konfiguriert ist, aufweist.

8. Ostomiebeutel (10) nach Anspruch 7, wobei die Drainagebefestigung (40) einen mindestens teilweise konischen Abschnitt beinhaltet.

9. Ostomiebeutel (10) nach Anspruch 7, ferner umfassend ein Sicherungsband (50) an der Drainagebefestigung (40), das zur Verbindung mit der Verschlussanordnung (26) konfiguriert ist.

10. Ostomiebeutel (10) nach Anspruch 1, ferner umfassend eine mit der Auslassvorrichtung verbundene Drainagebefestigung (40), wobei die Drainagebefestigung (40) einen inneren Verbindungsdurchgang (42), der mit dem inneren Durchgang (24) verbunden ist, ein erstes Segment (44) zum Ineingriffnehmen der Auslassvorrichtung (14), ein zweites Segment (46), das zur Verbindung mit einem Drainagesystem (40) konfiguriert ist, und ein Sicherungsband (50) aufweist, das dazu konfiguriert ist, die Verschlussanordnung (26) lösbar in Eingriff zu nehmen.

## Revendications

1. Poche pour stomie (10) comprenant :
une poche de collecte (12) comportant une cavité de collecte (20) définie en son sein et une ouverture (13) configurée pour recevoir une stomie ;
un dispositif de sortie (14) relié à la poche de collecte (12) et s'étendant à partir de celle-ci, le dispositif de sortie (14) définissant un passage interne (24) relié à la cavité de collecte (20), un agencement de fermeture (26) sur le dispositif de sortie (14) configuré pour refermer sélectivement le dispositif de sortie (14), dans laquelle l'agencement de fermeture (26) comprend une ou plusieurs lignes de pliage (30) dans le dispositif de sortie et une attache amovible (32), dans laquelle le dispositif de sortie (14) est configuré pour être replié sur lui-même le long de la ligne ou des lignes de pliage, et l'attache amovible (32) est configurée pour maintenir le dispositif de sortie (14) dans un état replié et fermé, **caractérisée en ce que** le dispositif de sortie est formé séparément de la poche de collecte et fixé à une sortie de poche de collecte, et **en ce que** le dispositif de sortie (14) et l'agencement de fermeture (26) sont formés d'un seul tenant comme une seule unité séparée de la poche de collecte (12).

2. Poche pour stomie (10) selon la revendication 1, dans laquelle le dispositif de sortie (14) est relié à la sortie de la poche de collecte par thermoscellage ou soudage, de telle sorte que le dispositif de sortie (14) est scellé à la sortie de la poche de collecte.

3. Poche pour stomie (10) selon la revendication 1, dans laquelle le dispositif de sortie (14) est moulé par injection comme une seule unité.

4. Poche pour stomie (10) selon la revendication 1, dans laquelle le dispositif de sortie (14) est moulé par soufflage.

5. Poche pour stomie (10) selon l'une quelconque des revendications précédentes, dans laquelle l'agencement de fermeture (26) comprend une seule ligne de pliage (30) et l'attache amovible (32) est une agrafe (34).

6. Poche pour stomie (10) selon l'une quelconque des revendications précédentes, dans laquelle l'agencement de fermeture (26) comprend deux lignes de pliage (30) et l'attache amovible (32) est l'un parmi une agrafe, un système de fermeture autoagrippante ou une pince.

7. Poche pour stomie (10) selon la revendication 1, comprenant en outre une fixation de drainage (40) ayant une première extrémité configurée pour être reçue dans le dispositif de sortie (14) et une seconde extrémité configurée pour être fixée à un tube de drainage (48).

8. Poche pour stomie (10) selon la revendication 7, dans laquelle la fixation de drainage (40) comprend une section au moins en partie conique.

9. Poche pour stomie (10) selon la revendication 7, comprenant en outre une sangle de fixation (50) sur la fixation de drainage (40) configurée pour être reliée à l'agencement de fermeture (26) .

10. Poche pour stomie (10) selon la revendication 1, comprenant en outre une fixation de drainage (40) reliée au dispositif de sortie, la fixation de drainage (40) comportant un passage de liaison interne (42) relié au passage interne (24), un premier segment (44) destiné à venir en prise avec le dispositif de sortie (14), un second segment (46) configuré pour être relié à un système de drainage (40), et une sangle de fixation (50) configurée pour venir en prise de manière amovible avec l'agencement de fermeture (26).
